# EUROPEAN PATENT APPLICATION

(11) **EP 4 606 887 A1**
(43) Date of publication of application: **27.08.2025**
(21) Application number: 23879808.6
(22) Date of filing: 18.10.2023
(51) Int. Cl.: C12N 5/071, C12N 7/00

(54) **CULTURE METHOD FOR FISH CELLS AND CELL CULTURE MEDIUM USED THEREIN, AND ORAL VACCINE FOR FISH**

(30) Priority: 19.10.2022 JP 2022167428
(71) Applicant: Biosiense Co., Ltd., Anan-shi, Tokushima 779-1292 (JP)
(72) Inventor: OKUTANI Asuka, Anan-shi, Tokushima 779-1292 (JP); SHUTO Kimihiro, Anan-shi, Tokushima 779-1292 (JP); KIMURA Yoshihiro, Anan-shi, Tokushima 779-1292 (JP); TANI Kosuke, Anan-shi, Tokushima 779-1292 (JP)
(74) Representative: Marks & Clerk LLP
(86) International application number: PCT/JP2023/037611
(87) International publication number: WO 2024/085165

(57) **Abstract**

The present disclosure provides a cell culture medium for use in culturing of fish cells, a method for culturing fish cells, and an oral vaccine for fish. In the present disclosure, the cell culture medium for use in culturing of fish cells preferably comprises serum obtained from adult fish.

## Description

### Technical Field

The present disclosure relates to a method for culturing fish cells, a cell culture medium for use in the method, and an oral vaccine for fish.

### Background Art

Animal cells are cultured for various purposes, and used in research and product manufacture. In general, animal cells of all species are cultured in the presence of fetal bovine serum. Fish cells are extensively cultured. Fish cells are also cultured in the presence of fetal bovine serum. In comparison with calf serum, fetal bovine serum is richer in growth factors, and contains cell growth inhibitory factors such as γ-globulin at a lower level (Non Patent Literature 1).

A method for culturing fish cells in the presence of fish serum is disclosed (Patent Literature 1). In Patent Literature 1, as a condition for fish from which the serum is derived, the fish is required to be sexually immature and in the logarithmic phase of growth.

### Citation List

### Patent Literature

Patent Literature 1: US 5426045 A

### Non Patent Literature

Non Patent Literature 1: Chelladurai et al., Heliyon, 7, e07686, 2021

### Summary of Invention

The present disclosure provides a method for culturing fish cells, a cell culture medium for use in the method, and an oral vaccine for fish. The present disclosure also provides a method for culturing a virus, a method for producing a virus, a cell culture medium for use in the methods, and an oral vaccine for fish.

According to the present disclosure, the following inventions are provided as an example.
(1) A composition for use in culturing of fish cells, comprising serum obtained from adult fish.
(2) A cell culture medium for use in culturing of fish cells, comprising serum obtained from adult fish.
(3) A method for culturing fish cells, comprising
   culturing fish cells in a cell culture medium containing serum obtained from adult fish.
(4) The method according to (3), wherein a concentration of the serum in the cell culture medium is 0.2% to 2%.
(5) A method for culturing a pathogen that infects fish, the method comprising:
   providing fish cells infected with the pathogen; and
   culturing the fish cells in a cell culture medium containing serum obtained from adult fish to proliferate the pathogen.
(6) The method according to (5), wherein a concentration of the serum in the cell culture medium is 0.2% to 2%.
(7) A method for preparing, from a pathogen that induces an infectious disease in fish, a vaccine against the infectious disease, the method comprising:
   providing fish cells infected with a pathogen that induces an infectious disease; and
   culturing the fish cells in a cell culture medium containing serum obtained from adult fish to proliferate the pathogen.
(8) The method according to (7), wherein a concentration of the serum in the cell culture medium is 0.2% to 2%.
(9) A composition comprising serum obtained from adult fish, for use in the method according to (3) or (4).
(10) A composition comprising serum obtained from adult fish, for use in the method according to any one of (5) to (8).

(1A) An oral inactivated vaccine for fish against a virus infectious disease, comprising an inactivated virus.
(2A) The oral inactivated vaccine for fish according to (1A), wherein the virus is an iridovirus.
(3A) The oral inactivated vaccine for fish according to (1A) or (2A), wherein a concentration of the inactivated virus is 1.0 × 10¹⁰ TCID₅₀/mL to 1.0 × 10¹² TCID₅₀/mL.
(4A) The oral inactivated vaccine for fish according to any one of (1A) to (3A), wherein an amount of the inactivated virus administered to fish individuals is 0.01 mL/day/fish to 1 mL/day/fish.
(5A) A method for treating fish, comprising
   orally administering to the fish an oral inactivated vaccine for fish against a virus infectious disease, which contains an inactivated virus, which imparts or induces immunity against the virus to the fish.
(6A) The method according to (5A), wherein the virus is an iridovirus.
(7A) The method according to (5A) or (6A), wherein the oral inactivated vaccine for fish is contained in feed.
(8A) A feed for fish, comprising an effective amount of the oral inactivated vaccine for fish according to any one of (1A) to (4A).
(9A) The feed for fish according to (8A), which is kneaded feed.

### Brief Description of Drawings

[Figure 1] Figure 1 shows a cell count of chicken grunt fin-derived cells on the ninth day of culturing thereof in the presence of adult yellowtail serum versus fetal bovine serum (FBS).
[Figure 2] Figure 2 shows a microscopic image of chicken grunt fin-derived cells on the fifth day of culturing thereof in the presence of adult yellowtail serum versus fetal bovine serum. The bar in the image indicates 200 µm.
[Figure 3] Figure 3 shows a cell count of chicken grunt fin-derived cells on the 35th day of culturing thereof in the presence of adult yellowtail serum or adult greater amberjack serum versus fetal bovine serum.
[Figure 4] Figure 4 shows a time-dependent change of the cell count of chicken grunt fin-derived cells in the presence of adult yellowtail serum versus fetal bovine serum (FBS).
[Figure 5] Figure 5 shows an estimated infectious titer of a red seabream iridovirus proliferated with chicken grunt fin-derived cells in the presence of adult yellowtail serum versus fetal bovine serum.
[Figure 6] Figure 6 shows a microscopic image of chicken grunt fin-derived cells on the fourth day after infection with the virus.
[Figure 7] Figure 7 shows a vaccine effect of a vaccine prepared from a red seabream iridovirus transmitted to chicken grunt fin-derived cells in the presence of adult yellowtail serum versus fetal bovine serum (FBS).
[Figure 8] Figure 8 shows a vaccine effect of an oral vaccine containing an inactivated iridovirus for yellowtail.
[Figure 9] Figure 9 shows a vaccine effect of an oral vaccine containing an inactivated iridovirus for red seabream.

### Detailed Description of Invention

In the present specification, the term "fish" refers to a group of animals which belong to Vertebrata and which are not four-footed animals. Examples of the fish include Hagfish, Lamprey, Chondrichthyes, and Osteichthyes. Osteichthyes is classified roughly into Sarcopterygii and Actinopterygii. Examples of Actinopterygii include Cladistia, Chondrostei, and Neopterygii. Examples of Neopterygii include Holostei and Teleostei. Examples of Teleostei include Cohort Elopomorpha such as Elopiformes, Notacanthiformes, and Anguilliformes; Osteoglossomorpha such as Osteoglossiformes and Hiodontiformes; Cohort Otocephala such as Clupeiformes, Alepocephaliformes, Gonorynchiformes, Cypriniformes, and Siluriformes; and Cohort Euteleostei such as Acanthopterygii, Osmeromorpha, Ateleopodomorpha, Cyclosquamata, Scopelomorpha, Lampridiomorpha, Paracanthopterygii, and Acanthopterygii. The fish is classified roughly into freshwater fish, saltwater fish, and fish that can live in both freshwater and seawater, according to a water regime where the fish grows. Examples of the fish that can live in both freshwater and seawater include seawater-oriented amphidromous fish, catadromous fish, anadromous fish, and amphidromous fish.

In the present specification, the "fish cell" is a cell isolated from fish. The fish cell can be cultured in the presence of fetal bovine serum. The fish cell is useful in, for example, production of vaccines against a pathogen on fish {in particular, farmed fish}. Examples of the farmed fish include yellowtail (young yellowtail), red seabream, greater amberjack, Pacific bluefin tuna, Japanese pufferfish, olive flounder, striped jack, Japanese jack mackerel, yellowtail amberjack, striped beakfish, thread-sail filefish, Japanese rockfish, marbled rockfish, Japanese black porgy, black rockfish, Japanese sea bass, crimson sea bream, chicken grunt, Japanese croaker, largescale blackfish, chub mackerel, seven band grouper, and long tooth grouper.

In the present specification, the "adult fish" is sexually matured fish. In the present specification, the fish before sexual maturation is called immature fish.

In the present specification, the "serum" is a supernatant of blood that has been clotted in the absence of an anticoagulant.

In the present specification, the term "culturing" means that fish cells are maintained or proliferated. The fish cells are cultured under conditions (nutrient conditions, temperature condition, atmospheric conditions, and the like) that are suitable for the culturing.

According to the present disclosure, a method for culturing fish cells and a composition for use in culturing of fish cells (for example, a cell culture medium) are provided. According to the present disclosure, fish cells are cultured in the composition for use in culturing of fish cells (for example, a cell culture medium) according to the present disclosure. The method for culturing fish cells according to the present disclosure involves conditions suitable for culturing of fish cells in addition to culturing of fish cells in the composition for use in culturing of fish cells (for example, a cell culture medium) according to the present disclosure. According to the present disclosure, an oral vaccine is also provided.

According to the present disclosure, fish cells can be cultured in the presence of serum obtained from fish (that is, in a composition or a cell culture medium containing serum obtained from fish). Examples of the fish include adult fish and immature fish. According to the present disclosure, in particular, fish cells can be cultured in the presence of serum obtained from immature fish, or preferably adult fish (that is, in a composition or a cell culture medium containing serum obtained from adult fish). In general, for animal cells, fetal bovine serum (FBS) is often used. Alternatively, as the serum, calf serum (CS) obtained from a calf after birth may be used. FBS has a higher ability to proliferate cells than CS, and is preferably used. For example, in comparison with CS, fetal bovine serum is more nutritious, and lower in content of gamma-globulin, immunoglobulin and complement factors that interfere with cell proliferation. CS contains lipids at a high concentration, but cell proliferation can be inhibited because of the high concentration of lipids. For such a reason, CS has been used for cells which are excessively proliferated in the presence of FBS. Thus, serum obtained from sexually mature cattle is normally not used in culturing of cells. The present disclosure includes the invention of being able to culture normal fish cells in the presence of serum obtained from adult fish. A certain aspect of the present disclosure provides the invention of being able to culture normal fish cells more efficiently in the presence of serum obtained from adult fish than in the presence of FBS.

According to a preferred aspect of the present disclosure, culturing does not require serum obtained from juvenile fish or immature fish. In a certain aspect, fish cells can be cultured in the presence of serum obtained from adult fish, instead of using serum obtained from juvenile fish or immature fish. In a certain aspect, the amount of use of serum obtained from juvenile fish or immature fish can be reduced by using serum obtained from adult fish. In a certain aspect, fish cells can be cultured in a medium containing, for example, 0.3% or less, 0.2% or less, or 0.1% or less of serum obtained from juvenile fish or immature fish, and serum obtained from adult fish. In a certain aspect, fish cells can be cultured in a medium which is free of serum obtained from juvenile fish or immature fish and which contains serum obtained from adult fish.

According to a preferred aspect of the present disclosure, fish cells can be cultured in the presence of serum obtained from immature fish (that is, in a composition or a cell culture medium containing serum obtained from fish). The immature fish is not limited, and can be any of the following fish, for example, Perciformes fish, for example, Percoidei fish, for example, Sparidae fish, in particular, red seabream. The immature fish is not limited, and can be Siluriformes fish, for example, Siluridae fish, in particular, Amur catfish.

According to Examples described later, the species of fish from which fish cells cultured are derived and the species of fish from which the serum is derived are in no way required to be identical. The cells can be cultured in all of the cases where (i) fish cells cultured are derived from saltwater fish and the serum is that of saltwater fish, (ii) fish cells cultured are derived from saltwater fish and the serum is derived from freshwater fish, (iii) fish cells cultured are derived from freshwater fish and the serum is that of saltwater fish, and (iv) fish cells cultured are derived from freshwater fish and the serum is derived from freshwater fish.

In a certain aspect, the fish from which the serum is derived is not limited, and can be any of the fish. In a certain aspect, the fish from which the serum is derived can be one or more fish belonging to a taxon selected from the group consisting of Hagfish, Lamprey, Chondrichthyes, and Osteichthyes. In a certain aspect, the fish from which the serum is derived can be one or more fish belonging to a taxon selected from the group consisting of Sarcopterygii and Actinopterygii. In a certain aspect, the fish from which the serum is derived can be one or more fish belonging to a taxon selected from the group consisting of Cladistia, Chondrostei, and Neopterygii. In a certain aspect, the fish from which the serum is derived can be one or more fish belonging to a taxon selected from the group consisting of Holostei and Teleostei. In a certain aspect, the fish from which the serum is derived can be one or more fish belonging to a taxon selected from the group consisting of Cohort Elopomorpha such as Elopiformes, Notacanthiformes, and Anguilliformes; Osteoglossomorpha such as Osteoglossiformes and Hiodontiformes; Cohort Otocephala such as Clupeiformes, Alepocephaliformes, Gonorynchiformes, Cypriniformes, and Siluriformes; and Cohort Euteleostei such as Acanthopterygii, Osmeromorpha, Ateleopodomorpha, Cyclosquamata, Scopelomorpha, Lampridiomorpha, Paracanthopterygii, and Acanthopterygii.

In a certain aspect, the fish from which the serum is derived is one or more selected from the group consisting of rainbow trout, eel, red spotted masu trout, Amur catfish, red stingray, spotless smooth-hound, chicken grunt, red seabream, yellowtail, Pacific bluefin tuna, and greater amberjack. In a certain aspect, the fish from which the serum is derived is one or more selected from the group consisting of rainbow trout, eel, red spotted masu trout, red stingray, spotless smooth-hound, chicken grunt, Pacific bluefin tuna, and yellowtail.

In a certain aspect, the fish from which the fish cells are derived is not limited, and can be any of fish. In a certain aspect, the fish from which the fish cells are derived can be one or more fish belonging to a taxon selected from the group consisting of Hagfish, Lamprey, Chondrichthyes, and Osteichthyes. In a certain aspect, the fish from which the fish cells are derived can be one or more fish belonging to a taxon selected from the group consisting of Sarcopterygii and Actinopterygii. In a certain aspect, the fish from which the fish cells are derived can be one or more fish belonging to a taxon selected from the group consisting of Cladistia, Chondrostei, and Neopterygii. In a certain aspect, the fish from which the fish cells are derived can be one or more fish belonging to a taxon selected from the group consisting of Holostei and Teleostei. In a certain aspect, the fish from which the fish cells are derived can be one or more fish belonging to a taxon selected from the group consisting of Cohort Elopomorpha such as Elopiformes, Notacanthiformes, and Anguilliformes; Osteoglossomorpha such as Osteoglossiformes and Hiodontiformes; Cohort Otocephala such as Clupeiformes, Alepocephaliformes, Gonorynchiformes, Cypriniformes, and Siluriformes; and Cohort Euteleostei such as Acanthopterygii, Osmeromorpha, Ateleopodomorpha, Cyclosquamata, Scopelomorpha, Lampridiomorpha, Paracanthopterygii, and Acanthopterygii.

In a certain aspect, the fish from which the fish cells are derived is farmed fish. In a certain aspect, the fish from which the fish cells are derived can be farmed fish selected from the group consisting of yellowtail (young yellowtail), red seabream, greater amberjack, Pacific bluefin tuna, Japanese pufferfish, olive flounder, striped jack, Japanese jack mackerel, yellowtail amberjack, striped beakfish, thread-sail filefish, Japanese rockfish, marbled rockfish, Japanese black porgy, black rockfish, Japanese sea bass, crimson sea bream, chicken grunt, Japanese croaker, largescale blackfish, chub mackerel, seven band grouper, long tooth grouper, and the like. In a certain aspect, the fish from which the fish cells are derived can be fish selected from the group consisting of yellowtail, red seabream, Pacific bluefin tuna, silver salmon, pufferfish, striped jack, and olive flounder. In a certain aspect, the fish from which the fish cells are derived can be fish selected from the group consisting of eel, salmon, trout, ayu, and carp.

In a certain aspect, the fish from which the fish cells are derived is wild fish. In a certain aspect, the fish from which the fish cells are derived can be wild fish selected from the group consisting of yellowtail and young yellowtail, red seabream, greater amberjack, Pacific bluefin tuna, Japanese pufferfish, olive flounder, striped jack, Japanese jack mackerel, yellowtail amberjack, striped beakfish, thread-sail filefish, Japanese rockfish, marbled rockfish, Japanese black porgy, black rockfish, Japanese sea bass, crimson sea bream, chicken grunt, Japanese croaker, largescale blackfish, chub mackerel, seven band grouper, long tooth grouper, and the like. In a certain aspect, the fish from which the fish cells are derived can be fish selected from the group consisting of yellowtail, red seabream, Pacific bluefin tuna, silver salmon, pufferfish, striped jack, and olive flounder. In a certain aspect, the fish from which the fish cells are derived can be fish selected from the group consisting of eel, salmon, trout, ayu, and carp.

In a certain aspect, the fish from which the fish cells are derived is one or more selected from the group consisting of chinook salmon, rainbow trout, bluegill, fathead minnow, striped snakehead, kuetama, chicken grunt, eel, red spotted masu trout, Amur catfish, red stingray, spotless smooth-hound, chicken grunt, red seabream, yellowtail and greater amberjack.

To the composition or cell culture medium for use in culturing of fish cells according to the present disclosure, serum obtained from fish (preferably adult fish) is added. In a certain aspect, the composition or cell culture medium for use in culturing of fish cells according to the present disclosure contains serum obtained from fish (preferably adult fish). In a certain preferred aspect, the composition or cell culture medium for use in culturing of fish cells according to the present disclosure contains serum obtained from adult fish. In a certain preferred aspect, the composition or cell culture medium for use in culturing of fish cells according to the present disclosure contains 0.2 to 5 vol%, 0.2 to 4 vol%, 0.2 to 3 vol%, 0.2 to 2 vol%, 0.2 to 1.9 vol%, 0.2 to 1.8 vol%, 0.2 to 1.7 vol%, 0.2 to 1.6 vol%, 0.2 to 1.5 vol%, 0.2 to 1.4 vol%, 0.2 to 1.3 vol%, 0.2 to 1.2 vol%, 0.2 to 1.1 vol%, or 0.2 to 1 vol% of serum obtained from adult fish. In a certain aspect, the composition or cell culture medium for use in culturing of fish cells according to the present disclosure contains serum obtained from adult fish, and may further contain serum obtained from juvenile fish or immature fish. In a certain aspect, the composition or cell culture medium for use in culturing of fish cells according to the present disclosure contains serum obtained from adult fish, and contains, for example, 0.3 vol% or less, 0.2 vol% or less, or 0.1 vol% or less of serum obtained from juvenile fish or immature fish. In a certain aspect, the composition or cell culture medium for use in culturing of fish cells according to the present disclosure contains serum obtained from adult fish, and is free of serum obtained from immature fish. The serum added is as described above. The medium contains a basal medium in addition to the serum. The basal medium typically contains a large number of components necessary for maintaining culture cells. Those skilled in the art will appreciate the types and components of media that can be used as the basal medium. The basal medium is not limited, and examples thereof include Dulbecco's modified Eagle medium (DMEM), minimum essential medium (MEM), Eagle's basal medium, DMEM/Ham's F12 medium, Leibovitz's L-15 medium, and Iscove's modified Dulbecco's medium. The medium can further contain antibiotics (for example, penicillin and streptomycin), L-glutamine, L-alanyl-L-glutamine, and the like.

According to the present disclosure, a method for reducing the amount of use of serum obtained from immature fish in culturing of fish cells, comprising adding serum obtained from adult fish to a medium to reduce the amount of use of the serum obtained from immature fish is also provided. According to the present disclosure, a method for avoiding use of serum obtained from immature fish in culturing of fish cells, comprising adding serum obtained from adult fish to a medium to avoid use of the serum obtained from immature fish is also provided. The serum added is as described above.

According to the present disclosure, a method for culturing a pathogen that infects fish is provided. The pathogen can be a virus that infects fish, pathogenic bacteria, or another pathogen. According to the present disclosure, the method for culturing a pathogen that infects fish comprises culturing fish cells infected with the pathogen in the composition or cell culture medium of the present disclosure. The method can comprise transmitting the pathogen to fish cells which the pathogen infects. The virus can be, for example, a herpesvirus, an iridovirus, a rhabdovirus, a reovirus, a birnavirus or another virus. These viruses have been reported to have infected fish. Examples of the herpesvirus include eel herpesvirus, goldfish hematopoietic necrosis virus), koi herpesvirus, flounder herpesvirus, lake trout EED, channel catfish herpesvirus, Salmonidae fish herpesvirus, white sturgeon herpesvirus type 1 (WSHV-1), white sturgeon herpesvirus type 2 (WSHV-2), and pilchard herpesvirus. Examples of the iridovirus include red seabream iridovirus, iridoviruses which cause epizootic hematopoietic necrosis or the like (Ranavirus including ISKNV, EHNV, ECV, ESV, and PPIV), iridoviruses which cause viral erythrocytic necrosis (VEN), sturgeon iridovirus, grouper iridovirus (Ranavirus), and iridoviruses which cause lymphocystis disease (LCDV) (lymphocystis disease virus). Examples of the rhabdovirus include rhabdoviruses which cause infectious hematopoietic necrosis (IHN) (infectious hematopoietic necrosis virus; IHNV), eel virus American (EVA), snakehead rhabdovirus, perch rhabdovirus, flounder rhabdovirus, and red seabream VHS. Examples of the reovirus include fish orthoreovirus, and aquareovirus. Examples of the birnavirus include birnaviruses which cause infectious pancreatic necrosis (infectious pancreatic necrosis virus; IPNV), birnaviruses which cause viral deformity, and birnaviruses which cause viral ascites. Examples of the other virus include viruses which cause (ayu) atypical cellular gill disease (Plecoglossus altivelis poxvirus; PaPV), viruses which cause viral carp edema (carp edema virus; CEV), viruses which cause viral nervous necrosis (VNN), viruses which cause (eel) viral endothelial cell necrosis, viruses which cause eel picornavirus-1 infectious disease (EPV-1), viruses which cause chinook salmon paramyxovirus, viruses which cause infectious salmon anemia (ISA), viruses which cause tilapia lake virus (TiLV), baculoviruses which cause shrimp baculovirus midgut gland necrosis (BMNV), and viruses which cause white spot disease in which a body color change and formation of white spots in the outer skeleton are observed in the shrimp (WSV).

According to the present disclosure, from a pathogen that has been cultured, a vaccine against the pathogen can be prepared. Examples of the vaccine include live vaccine and inactivated vaccine. The live vaccine is vaccine in which the relevant pathogenicity is suppressed to a level at which a symptom does not develop (the attenuated pathogen is used as vaccine). The inactivated vaccine is vaccine prepared by heating a pathogen or treating the pathogen with a chemical such as formalin (that is, inactivating the pathogen) to eliminate the relevant pathogenicity. According to the present disclosure, a method for preparing, from a pathogen that induces an infectious disease in fish, a vaccine against the infectious disease is provided. The method for preparing the vaccine can comprise providing fish cells infected with a pathogen that induces an infectious disease, and culturing the fish cells in a cell culture medium containing serum obtained from adult fish to proliferate the pathogen. The fish cells are cultured in the medium under conditions suitable for the culturing. The method for preparing the vaccine may comprise attenuating the obtained pathogen, or inactivating the obtained pathogen to obtain an inactivated vaccine.

In an aspect, the vaccine can be orally administered. Accordingly, the present disclosure provides an oral vaccine (that is, a vaccine that is orally administered or a vaccine for oral administration). In a certain preferred aspect, the vaccine can contain an effective amount of an inactivated virus. In a certain preferred aspect, the vaccine can contain an effective amount of an inactivated iridovirus (preferably an inactivated red seabream iridovirus). The vaccine can be complete particle vaccine or split vaccine, preferably complete particle vaccine. In a certain preferred aspect, the virus can be an inactivated VNN virus. The target of oral administration can be fish individuals, preferably farmed fish individuals.

In a certain aspect, the vaccine can contain (i) an inactivated product of a virus, or (ii) an inactivated product of virus-producing cells and a culture supernatant of the cells. The inactivated product of a virus is obtained in the manner described above. The virus is transmitted to cells that are cultured in the presence of fetal bovine serum (FBS), or preferably fish serum. When the virus transmitted to the cells sufficiently proliferates, the virus can be collected. The inactivated product of virus-producing cells and a culture supernatant of the cells can be obtained by collecting a medium containing cells which are infected with a virus and are producing the virus, followed by inactivation with, for example, formalin.

The inactivated product is preferably concentrated by centrifugation or the like. In a certain aspect, the inactivated product can be concentrated 50-fold or more, 60-fold or more, 70-fold or more, 80-fold or more, 90-fold or more, preferably 100-fold or more, more preferably 110-fold or more, further more preferably 120-fold or more, even more preferably 130-fold or more, particularly preferably 140-fold or more, most preferably 150-fold or more, or about 160-fold to, for example, 1.0 × 10¹⁰ to 1.0 × 10¹² TCID₅₀/mL, for example, 2.0 × 10¹⁰ to 5.0 × 10¹¹ TCID₅₀/mL, for example, 3.0 × 10¹⁰ to 3.0 × 10¹¹ TCID₅₀/mL, preferably 4.0 × 10¹⁰ to 1.6 × 10¹¹ TCID₅₀/mL. The concentration can be performed for reducing the moisture content or removing moisture. In a certain aspect, the concentration can be performed for reducing the moisture content or removing moisture to the extent that there is no inconvenience to subsequent mixing of the inactivated product with food, maintenance of the shape of the obtained food, and the like. However, reduction of the moisture content after mixing with food is not excluded. The concentrated inactivated product can be used as vaccine. The vaccine can contain the concentrated inactivated product. The vaccine can include the concentrated inactivated product, and an attached document about the vaccine.

The concentrated inactivated product can be stored at 2°C to 8°C, preferably 4°C. Alternatively, the concentrated inactivated product can be stored in a frozen state.

The oral vaccine can be mixed with feed for fish (fishery feed, farming feed, or the like) before administration to fish. The feed for fish can be appropriately selected according to fish to be given the feed. The mixing ratio of the vaccine and the feed can be appropriately determined. For example, the amount of the vaccine (the amount of the concentrated inactivated product) with respect to the mixture can be set to 1 wt% to 50 wt%. A thickener (for example, a polysaccharide thickener) such as guar gum may be further added in mixing of the vaccine with the feed. Examples of the thickener include thickeners including polysaccharide thickeners, and more specific examples thereof include carrageenan, carboxymethyl cellulose, xanthan gum, guar gum, and pectin. By adding the thickener, the shape of the feed mixed with the vaccine can be stabilized, and for example, the shape is easily maintained in water. Therefore, for example, the thickener in an amount sufficient to stabilize the shape of the feed, or an amount sufficient to stabilize the shape of the feed in water can be mixed with the vaccine and the feed. The feed mixed is not limited, and can be kneaded feed. Fish individuals freely take the food, resulting in obtainment of a sufficient vaccine effect. Thus, according to the present disclosure, a feed for fish (for example, kneaded feed), comprising an effective amount of the inactivated virus is provided.

The amount of the inactivated virus administered to fish individuals can be, for example, 0.01 to 1 mL/day/fish, preferably 0.02 to 0.7 mL/day/fish, preferably 0.03 to 0.5 mL/day/fish, preferably 0.04 to 0.4 mL/day/fish, preferably 0.05 to 0.3 mL/day/fish, more preferably 0.06 to 0.25 mL/day/fish.

The virus concentration in the inactivated product can be, for example, 1.0 × 10¹⁰ to 1.0 × 10¹² TCID₅₀/mL, for example, 2.0 × 10¹⁰ to 5.0 × 10¹¹ TCID₅₀/mL, for example, 3.0 × 10¹⁰ to 3.0 × 10¹¹ TCID₅₀/mL, preferably 4.0 × 10¹⁰ to 1.6 × 10¹¹ TCID₅₀/mL.

The dose of the inactivated virus administered to fish individuals can be, for example, 5.0 × 10⁹ to 1.0 × 10¹¹ TCID₅₀/day/fish, preferably 1.0 × 10¹⁰ to 5.0 × 10¹⁰ TCID₅₀/day/fish.

According to the present disclosure, a method for feeding fish individuals, comprising making the fish individuals take food containing an effective amount of the oral vaccine is provided. In this method, the fish individuals take the food to induce immunity against a virus in the body. Thus, according to the present disclosure, a method for inducing immunity against a virus to fish individuals, comprising making the fish individuals take the oral vaccine is provided. In this method, the oral vaccine may be contained in the food. Thus, according to the present disclosure, a feed for fish (for example, kneaded feed), containing an inactivated virus is provided. The virus can be preferably an iridovirus. In a certain preferred aspect, the virus can be an inactivated VNN virus.

According to the present disclosure, an oral vaccine for use in the above-described method (that is, an oral vaccine containing an inactivated virus, for use in a method for inducing immunity against a virus to fish individuals, comprising making the fish individuals take the oral vaccine) is provided. According to the present disclosure, virus-producing cells containing a virus (and preferably a solid content in a culture supernatant), for use in the above-described method is also provided. The collected virus-producing cells containing a virus and preferably the collected solid content in the culture supernatant (which is a solid content of a culture containing the virus and cells) can be inactivated by formalin treatment or the like. The solid content in the culture supernatant can be a solid component (containing, for example, a virus or inactivated virus) which can be precipitated by centrifugation. The solid content of the culture containing the virus and cells can be blended in feed for fish (for example, kneaded feed). Thus, according to the present disclosure, a feed for fish (for example, kneaded feed), containing a solid content of a culture containing an inactivated virus and cells is provided. The virus can be preferably an iridovirus. In a certain preferred aspect, the virus can be an inactivated VNN virus. The feed can be given to fish for feeding and vaccination.

According to the present disclosure, a method for producing a vaccine which is orally administered to fish individuals can comprise infecting fish cells with a virus under appropriate conditions in a medium containing adult fish serum, and culturing the virus-infected cells to proliferate the virus in the cells. According to the present disclosure, the method may further comprise performing inactivation treatment on a culture containing the virus and obtained by the culturing. The virus is also contained in the cells, and the production amount (yield) of the inactivated virus can be increased by performing the inactivation treatment on the cells containing the virus. Here, it is not necessary to release the virus by disrupting the cells in advance by a physical method or the like. Particularly, in the case of an iridovirus, it may be effective to perform inactivation treatment on cells because the virus is present in a large amount in the cells. According to the present disclosure, an inactivated virus preparation containing a virus at a high concentration can be prepared. The inactivated virus preparation preferably has undergone concentration treatment. The concentration treatment can be performed before or after the inactivation treatment, preferably after the inactivation treatment, and enables the moisture content to be reduced to a sufficient degree for mixing with the food, for example. The concentration treatment is not limited, and can be, for example, centrifugation treatment or filtration through a filter. It is not necessary to remove cells or fragments thereof by the concentration treatment, and the concentration treatment may be centrifugation treatment. It is desirable that the concentration treatment allow the shape of food to be easily maintained after the preparation and the food are mixed. In the case where it is necessary to add a thickener for maintaining the shape, the thickener can be appropriately added.

According to the present disclosure, there is provided a method for producing a food containing an inactivated vaccine which is orally administered to fish individuals, the method comprising mixing the oral vaccine and food (in particular, feed for fish). According to the present disclosure, there is provided a method for producing a vaccine which is orally administered to fish individuals, the method comprising mixing the oral vaccine, food and a thickener. The food is preferably kneaded feed. By allowing the fish individuals to freely take the food containing the vaccine, immunity against a virus can be induced in the fish individuals.

### Examples

### Example 1: Culturing of fish-derived cells using fish serum-containing medium

In the present Example, fish-derived cells were cultured in a medium containing serum derived from sexually mature fish.

### Method

Using a cell culture medium (Dulbecco's modified Eagle medium) to which fetal bovine serum (hereinafter FBS) had been added at a final concentration of 10%, chicken grunt fin-derived cells (hereinafter GF cells) were cultured in a closed environment at 25°C. The culture supernatant of GF cells during culturing was removed, the cells were then washed once with 10 mL of phosphate buffer saline (hereinafter PBS), and 1.0 mL of a trypsin/EDTA solution was added to suspend the cells. To the cell suspension, 9 mL of the cell culture medium was added, and the cells were sufficiently suspended, followed by centrifugation. After the centrifugation, the supernatant was removed. In a 25 cm² flask containing 4 mL of a cell culture medium to which FBS or adult yellowtail serum (treated for inactivation at 45°C for 20 minutes) had been added at 10%, 5%, 1% or 0.3%, the cells were seeded at 0.43 × 10⁶ cells/flask. The adult yellowtail serum had been obtained from adult yellowtail confirmed to be sexually mature. As a control, in a 25 cm² flask containing 4 mL of a cell culture medium free of serum (hereinafter a serum-free medium), the cells were seeded at 0.43 × 10⁶ cells/flask. The cells were cultured in a closed environment at 25°C for 9 days, and cell counting was performed on the zeroth day of culturing (at cell seeding time) and on the ninth day of culturing.

### Results

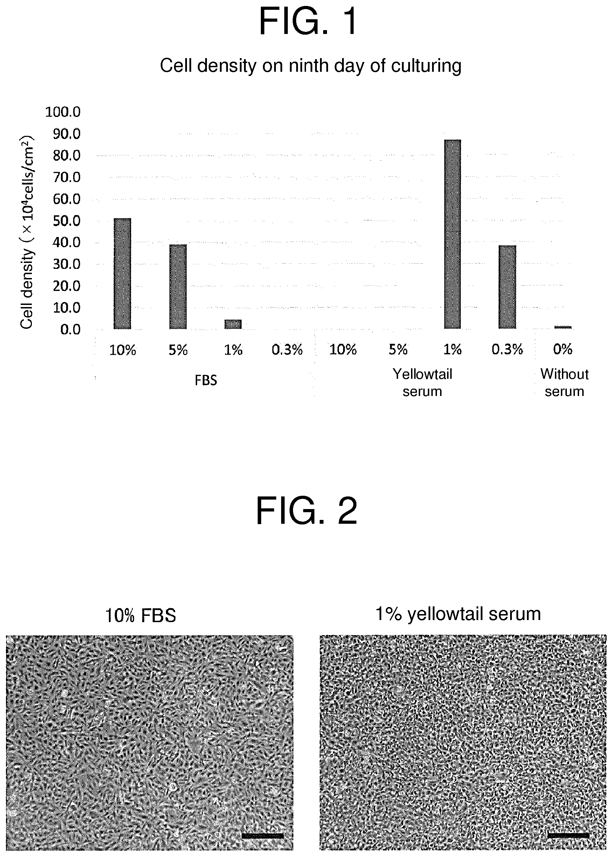

In the group with a 10%, 5%, 1% or 0.3% FBS-containing cell culture medium, the group with a 1% or 0.3% adult yellowtail serum-containing cell culture medium, and the group with a serum-free medium, the GF cells were fixed on the first day of culturing, whereas in the groups with 10% and 5% adult yellowtail serum-containing cell culture media, the fixation did not occur. In the groups with 10% and 5% FBS-containing cell culture media and 1% and 0.3% adult yellowtail serum-containing cell culture media, the cells proliferated to 51.2 × 10⁴ cells/cm², 39.0 × 10⁴ cells/cm², 87.0 × 10⁴ cells/cm² and 38.4 × 10⁴ cells/cm², respectively, on the ninth day of culturing as shown in Figure 1. As shown in Figure 2, a difference in the number of cells and cell density became obvious under a microscope on the fifth day of culturing. The number of cells in the group with a 1% adult yellowtail serum-containing cell culture medium was larger than that in the group with a 10% FBS-containing cell culture medium (see Table 1, and Figures 1 and 2). On the other hand, in the groups with 1% and 0.3% FBS and 10% and 5% adult yellowtail serum-containing cell culture media, little or no cell proliferation was observed.

**[Table 1]**

| Table 1: Cell density on ninth day of culturing | | | |
|---|---|---|---|
| No. | Serum | Concentration added | Cell density (× 10⁴ cells/cm²) |
| 1 | FBS | 10% | 51.2 |
| 2 | | 5% | 39.0 |
| 3 | | 1% | 4.6 |
| 4 | | 0.3% | 0.0 |
| 5 | Adult yellowtail serum | 10% | 0.0 |
| 6 | | 5% | 0.0 |
| 7 | | 1% | 87.0 |
| 8 | | 0.3% | 38.4 |
| 9 | Without serum | 0% | 1.3 |

### Example 2: Culturing of fish-derived cells using fish serum-containing medium (long-period culturing)

In the present Example, proliferation of cells in long-period culturing in the presence of fish serum was observed.

Using a cell culture medium (Dulbecco's modified Eagle medium) to which FBS had been added at 10%, chicken grunt fin-derived cells (hereinafter GF cells) were cultured in a closed environment at 25°C. The culture supernatant of GF cells during culturing was removed, the cells were then washed once with 10 mL of phosphate buffer saline (hereinafter PBS), and 1.0 mL of a trypsin/EDTA solution was added to suspend the cells. To the cell suspension, 9 mL of the cell culture medium was added, and the cells were sufficiently suspended. Thereafter, in a 25 cm² flask containing 4 mL of a cell culture medium to which adult yellowtail serum (treated for inactivation at 45°C for 20 minutes) or adult greater amberjack serum (treated for inactivation at 45°C for 20 minutes) had been added at 1%, 0.5%, 0.3% or 0.1%, the cells were seeded at 0.43 × 10⁶ cells/flask. The adult yellowtail serum and the adult greater amberjack serum had been obtained, respectively, from adult fish of yellowtail and greater amberjack confirmed to be sexually mature. As a control, in a 25 cm² flask containing 4 mL of a 10% FBS-containing cell culture medium which contains 10% of FBS, the cells were seeded at 0.43 × 10⁶ cells/flask. The cells were cultured in a closed environment at 25°C for 35 days, and cell counting was performed on the zeroth day of culturing (at cell seeding time) and on the 35th day of culturing. Further, in addition to the above, a group was provided in which the cells were cultured in a serum-free medium until cell fixation, and then in a serum-containing medium. Specifically, the cells were cultured in a serum-free medium until the day after seeding, and the cells were fixed on the culture surface of the flask. After the cell fixation, the cells were cultured in cell culture media containing 1%, 0.5%, 0.3% and 0.1% of adult yellowtail serum or adult greater amberjack serum, or 10% of FBS.

### Results

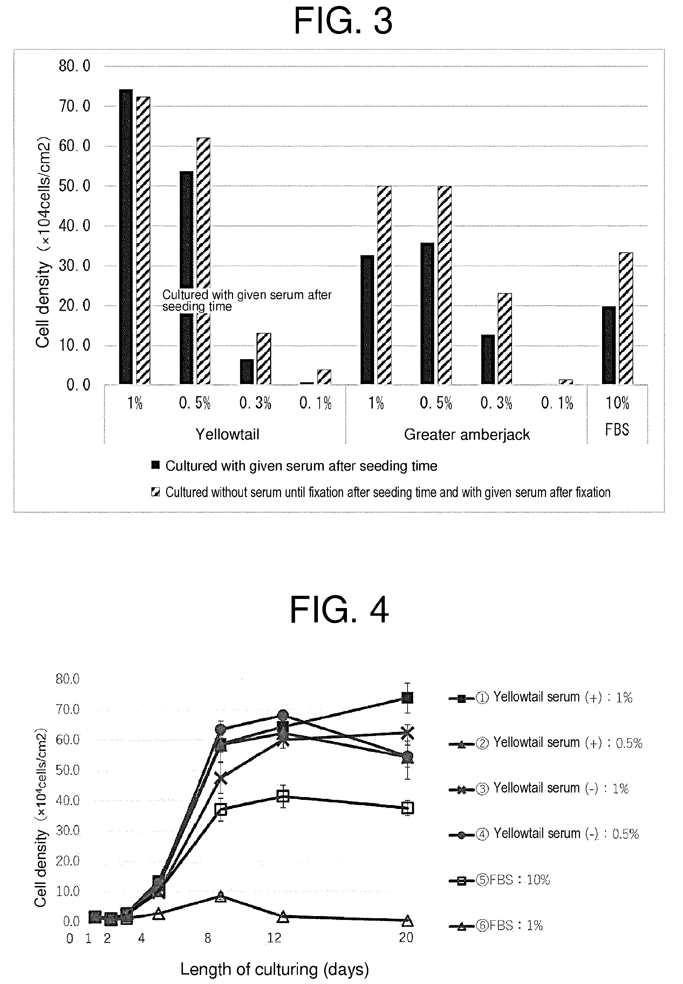

Both the group with an adult yellowtail serum-containing cell culture medium and the group with an adult greater amberjack serum-containing cell culture medium had a higher cell density over the group with a 10% FBS-containing cell culture medium at an addition concentration of 0.5% or more (see Table 2 and Figure 3). Both the group with an adult yellowtail serum-supplemented cell culture medium and the group with an adult greater amberjack serum-containing cell culture medium tended to have a low cell density at an addition concentration of 0.3% or less. In particular, when the cells were cultured with adult yellowtail serum added at a concentration of 1%, the cell density was 74.2 × 10⁴ cells/cm², and in the group with FBS added at 10%, the cell density was 10.8 × 10⁴ cells/cm². Comparison shows that the number of cells was 3.75 times larger in the culturing with adult yellowtail serum added at 1%. A similar tendency was recognized when the cells were cultured in a serum-free medium at the time of cell fixation, and both the group with an adult yellowtail serum-supplemented cell culture medium and the group with an adult greater amberjack serum-containing cell culture medium had a higher cell density over the group with a 10% FBS-containing cell culture medium at an addition concentration of 0.5% or more, and tended to have a low cell density at an addition concentration of 0.3% or less.

**[Table 2]**

| Table 2: Culture density on 35th day of culturing | | | | |
|---|---|---|---|---|
| No. | Serum | Concentration added | Cell density (× 10⁴ cells/cm²) | |
| | | | Cultured with given serum after seeding time | Cultured without serum until fixation after seeding time and with given serum after fixation |
| 1 | Adult yellowtail serum | 1% | 74.2 | 72.3 |
| 2 | | 0.5% | 53.8 | 62.1 |
| 3 | | 0.3% | 6.6 | 13.1 |
| 4 | | 0.1% | 0.8 | 3.8 |
| 5 | Adult greater amberjack serum | 1% | 32.6 | 49.9 |
| 6 | | 0.5% | 35.8 | 49.9 |
| 7 | | 0.3% | 12.7 | 23.0 |
| 8 | | 0.1% | 0.2 | 1.3 |
| 9 | FBS | 10% | 19.8 | 33.3 |

### Example 3: Test for confirmation of ability to proliferate fish-derived cells using fish serum-containing medium

In the present Example, proliferation of cells in the presence of fish serum was confirmed over time.

### Method

Using a cell culture medium (Dulbecco's modified Eagle medium) to which FBS had been added at a final concentration of 10%, chicken grunt fin-derived cells (hereinafter GF cells) were cultured in a closed environment at 25°C. The culture supernatant of GF cells during culturing was removed, the cells were then washed once with 10 mL of phosphate buffer saline (hereinafter PBS), and 1.0 mL of a trypsin/EDTA solution was added to suspend the cells. To the cell suspension, 9 mL of the cell culture medium was added, and the cells were sufficiently suspended. Thereafter, in a 25 cm² flask containing 4 mL of a cell culture medium to which adult yellowtail serum (treated for inactivation at 45°C for 20 minutes; described as "adult yellowtail serum (+)" in Table 3) or adult yellowtail serum (not treated for inactivation; described as "adult yellowtail serum (-)" in Table 3) had been added at 1% or 0.5%, the cells were seeded at 0.43 × 10⁶ cells/flask. The adult yellowtail serum had been obtained from adult yellowtail confirmed to be sexually mature. As a control, in a 25 cm² flask containing 4 mL of a 10% FBS or 1% FBS-containing cell culture medium which contains 10% or 1% of FBS, the cells were seeded at 0.43 × 10⁶ cells/flask. The cells were cultured in a closed environment at 25°C for 20 days, and cell counting was performed on the first, second, fourth, eighth, 12th and 20th day after the start of culturing.

### Results

For the adult yellowtail serum, regardless of whether the serum had been treated for inactivation or had not been treated for inactivation, a maximum cell density was recognized on and after the 12th day after culturing (see Table 3 and Figure 4). Comparison of the maximum cell density between the group with a 10% FBS-containing culture medium and the group with an adult yellowtail serum-containing cell culture medium showed that the maximum cell density was 4.7 × 10⁵ cells/cm² in the former and 6.8 × 10⁵ cells/cm² to 7.8 × 10⁵ cells/cm² in the latter, and thus, the cells cultured with adult yellowtail serum had a higher maximum cell density. In addition, when the group with an adult yellowtail serum-containing cell culture medium and the group with a FBS-containing culture medium were compared for the proliferation rate between the fourth day and the eighth day, it was confirmed that the group with an adult yellowtail serum-containing cell culture medium obviously had shorter doubling time.

**[Table 3]**

| Table 3: Cell density of GF cells cultured with given serum at given concentration | | | | | | | | | | |
|---|---|---|---|---|---|---|---|---|---|---|
| Test group | | Length of culturing (days) | | | | | | | Doubling time on fourth to eighth day of culturing (h) | Maximum density (×10⁴ cells/cm²) |
| | | 0 | 1 | 2 | 4 | 8 | 12 | 20 | | |
| 1% Adult yellowtail serum (+) | Total cell density (×10⁴cells/cm²) | 0.0 | 0.7 | 2.8 | 13.4 | 58.7 | 64.3 | 73.6 | 45.2 | 73.6 |
| | S.D. | - | 0.1 | 0.2 | 1.4 | 2.7 | 4.4 | 4.9 | | |
| 0.5% Adult yellowtail serum (+) | Total cell density (×10⁴cells/cm²) | 0.0 | 1.0 | 2.1 | 11.9 | 58.4 | 62.1 | 54.1 | 41.9 | 62.1 |
| | S.D. | - | 0.1 | 0.3 | 0.5 | 5.6 | 2.7 | 7.3 | | |
| 1% Adult yellowtail serum (-) | Total cell density (×10⁴cells/cm²) | 0.0 | 0.8 | 2.5 | 9.8 | 47.5 | 60.0 | 62.1 | 42.2 | 62.1 |
| | S.D. | - | 0.0 | 0.2 | 0.4 | 5.1 | 2.8 | 2.7 | | |
| 0.5% Adult yellowtail serum (-) | Total cell density (×10⁴cells/cm²) | 0.0 | 0.6 | 2.4 | 12.6 | 63.5 | 68.0 | 54.4 | 41.1 | 68.0 |
| | S.D. | - | 0.0 | 0.7 | 0.5 | 2.7 | 1.3 | 3.6 | | |
| 10% FBS | Total cell density (×10⁴cells/cm²) | 0.0 | 1.1 | 2.6 | 10.2 | 37.1 | 41.3 | 37.3 | 51.7 | 41.3 |
| | S.D. | - | 0.1 | 0.3 | 1.1 | 3.7 | 3.7 | 2.5 | | |
| 1% FBS | Total cell density (×10⁴cells/cm²) | 0.0 | 0.7 | 1.2 | 2.7 | 8.4 | 1.6 | 0.2 | 58.8 | 8.4 |
| | S.D. | - | 0.2 | 0.0 | 0.2 | 1.1 | 0.0 | 0.0 | | |

### Example 4: Viral culture test using fish serum-containing medium

In the present Example, a virus was cultured using fish cells cultured in the presence of fish serum.

### Method

### Culturing of red seabream iridovirus

Using a cell culture medium (Dulbecco's modified Eagle medium) to which FBS had been added at 10%, chicken grunt fin-derived cells (hereinafter GF cells) were cultured in a closed environment at 25°C. The culture supernatant of GF cells during culturing was removed, the cells were then washed once with 10 mL of phosphate buffer saline (hereinafter PBS), and 1.0 mL of a trypsin/EDTA solution was added to suspend the cells. To the cell suspension, 9 mL of the cell culture medium was added, and the cells were sufficiently suspended, followed by centrifugation. After the centrifugation, the supernatant was removed. In a 25 cm² flask containing 4 mL of a cell culture medium to which adult yellowtail serum (treated for inactivation at 45°C for 20 minutes) or adult greater amberjack serum (treated for inactivation at 45°C for 20 minutes) had been added at 1%, 0.5%, 0.3% or 0.1%, the cells were seeded at 0.43 × 10⁶ cells/flask. The adult yellowtail serum and the adult greater amberjack serum had been obtained, respectively, from adult fish of yellowtail and greater amberjack confirmed to be sexually mature. As a control, in a 25 cm² flask containing 4 mL of a 10% FBS-containing cell culture medium which contains 10% of FBS, the cells were seeded at 0.43 × 10⁶ cells/flask. The cells were cultured in a closed environment at 25°C. With the cells being in a state of monolayer, red seabream iridovirus BI11 strain was inoculated at 1.0 × 10⁷ TCID₅₀/cm² and transmitted to the cells, which were cultured until the cytopathic effect (CPE) extended across all the cells. After completion of the culturing, the culture was collected, and used as a red seabream iridovirus culture solution.

### Measurement of estimated infectious titer using real-time PCR

The red seabream iridovirus culture solution with a known concentration (3.2 × 10⁷ TCID₅₀/mL) was serially diluted 10-fold with the cell culture medium to obtain a standard sample, and a culture solution of the red seabream iridovirus cultured with given serum was diluted 1,000 fold with the cell culture medium to obtain a measurement sample. DNA purification was performed on each sample using an InstaGene DNA purification matrix (Bio-Rad Laboratories, Inc.), and the resulting product was used for real-time PCR. A calibration curve was prepared from the Ct value of the standard sample, and an estimated infectious titer (TCID₅₀/mL) was calculated from the Ct value of each measurement sample.

### Results

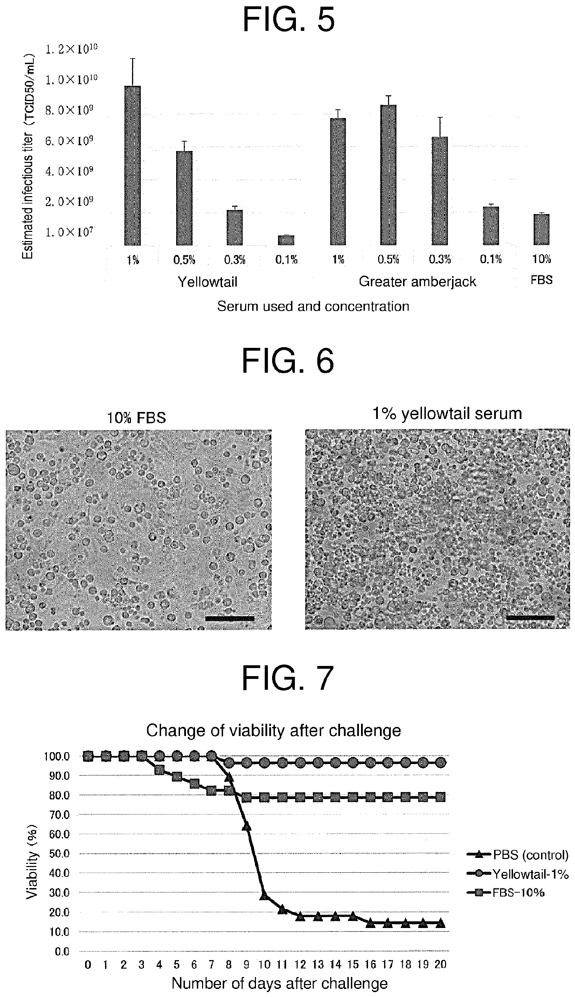

The red seabream iridovirus cultured in the adult yellowtail serum-containing cell culture medium and the adult greater amberjack serum-containing cell culture medium each having a serum addition concentration of 0.3% or more had an estimated infectious titer of 2.2 × 10⁹ to 9.7 × 10⁹ TCID₅₀/mL (see Figure 5). Such an estimated infectious titer was higher than the estimated infectious titer of the red seabream iridovirus cultured in the 10% FBS-containing cell culture medium, which is 1.9 × 10⁹ TCID₅₀/mL (see Figure 5). Comparison of the infectious titer showed that the former infectious titer was 1.2 to 5.1 times the latter. A difference in proportion of CPE resulting from virus infection was obvious under a microscope (see Figure 6), and virus culturing with adult fish serum was thus confirmed to provide a large number of virus particles with a smaller addition amount of serum which is equal to or less than 1/10 times that of FBS, and with good efficiency.

### Example 5: Test of safety and confirmation of effectiveness of viral vaccine prepared using fish serum for fish

In the present Example, an inactivated vaccine was prepared from a virus proliferated by culturing fish cells in the presence of adult fish serum, and the effectiveness and the safety of the prepared vaccine were confirmed.

### Method

For confirming that a prototype vaccine prepared using adult fish serum was safe for animal subjects, and effective as vaccine against challenge with a red seabream iridovirus, the prototype vaccine was injected to juvenile yellowtail to confirm the safety. In addition, an artificial infection test was conducted by injecting a red seabream iridovirus solution to yellowtail inoculated with the prototype vaccine in advance, and the immune effect against the red seabream iridovirus was confirmed.

### Method for preparing prototype vaccine

Formalin was added at a final concentration of 0.2 vol% to each of a culture solution of the red seabream iridovirus cultured using 1% adult yellowtail serum and a culture solution of the red seabream iridovirus cultured using 10% FBS, which had been prepared in Example 4 above, followed by inactivation at 4°C for 14 days. The inactivated red seabream iridovirus culture solution adjusted to a pH of 7.5 was used as a prototype red seabream iridovirus vaccine.

### Immunization method

The yellowtail farmed in a 100 L tank at 28 fish per tank was intraperitoneally injected with 0.1 mL of the prototype red seabream iridovirus vaccine cultured using 1% adult yellowtail serum or 10% FBS. A control group (PBS group) was injected with 0.1 mL of PBS. After the injection, the fish was farmed at a water temperature of 27°C for 9 days, whether or not there was an abnormal swimming pattern in the farming period was confirmed, and the number of dead fish was counted. The fish body weight was measured at the start of immunization and at the end of immunization.

### Challenge method

After completion of the immunization, 0.1 mL of challenging red seabream iridovirus solution BI6 strain was intraperitoneally injected at 4.0 × 10¹ TCID₅₀/fish. After the challenge, the fish was farmed at a water temperature of 27°C for 20 days, and the viability and the mortality in each test group were calculated. For the viability, a significant difference was calculated by the Fisher's exact test method between the test groups and the control group (p < 0.05).

### Results

After the vaccine administration (during immunity period), all the test groups exhibited a good weight gain (Table 4-1). After the vaccine administration, death did not occur during the immunity period and there was no abnormal finding in clinical observation in any of the test groups (Table 4-2). From this, it was confirmed that preparation of a prototype viral vaccine with fish serum, followed by injection of the vaccine to fish, was safe for the fish as in the case of preparation with FBS.

Next, the effect of the vaccination against the challenge was examined. The number of fish died after the challenge is shown in Table 4-3, and a change of the viability is shown in Figure 7. In the PBS group, death occurred on and after the eighth day of challenge, with the mortality being 85.7% and the viability being 14.3% at the end. On the other hand, the yellowtail serum group and the FBS group had mortalities of 3.6% and 21.4%, respectively, viabilities of 96.4% and 78.6%, respectively, and vaccine effectiveness percentages of 95.8% and 75.0%, respectively. Both the protocol vaccines were confirmed to have a viability significantly higher than that in the PBS group (p < 0.05). The value of the effectiveness percentage in the yellowtail serum group was higher than that in the FBS group, and the vaccine with yellowtail serum was confirmed to be more effective.

**[Table 4-1]**

| Table 4-1: Average fish body weight and gain of body weight during period of immunization | | | | | |
|---|---|---|---|---|---|
| Test group | Start of immunization | | End of immunization | | Gain of body weight (g) |
| | Average fish body weight (g) | S.D. | Average fish body weight (g) | S.D. | |
| PBS | 5.9 | 1.4 | 7.6 | 1.3 | 1.7 |
| Adult yellowtail serum | 6.0 | 1.0 | 7.5 | 1.4 | 1.5 |
| FBS | 6.1 | 1.2 | 7.2 | 1.7 | 1.1 |

**[Table 4-2]**

| Table 4-2: Number of fish died after vaccination | | | | |
|---|---|---|---|---|
| Test group | Number of fish tested (number of fish) | Mortality (%) (dead/tested) | Viability (%) (alive/tested) | Clinical finding |
| PBS | 28 | 0.0 (0/28) | 100.0 (28/28) | No abnormal finding ^{*1} |
| Adult yellowtail serum | 28 | 0.0 (0/28) | 100.0 (28/28) | No abnormal finding^{*1} |
| FBS | 28 | 0.0 (0/28) | 100,0 (28/28) | No abnormal finding ^{*1} |

| | | | | |
|---|---|---|---|---|
| *1 No abnormal finding: Abnormality was not observed in the body color, the swimming pattern of fish schools and the feeding behavior in nine days of clinical observation after administration. | | | | |

**[Table 4-3]**

| Table 4-3: Number of fish died after challenge | | | | | |
|---|---|---|---|---|---|
| Test group | Number of fish tested (number of fish) | Mortality (%) (dead/tested) | Viability (%) (alive/tested) | Effectiveness percentage (%) | Significant difference (p-value; %) |
| PBS | 28 | 85.7 (24/28) | 14.3 (4/28) | - | - |
| Adult yellowtail serum | 28 | 3.6 (1/28) | 96.4 (27/28) | 95.8 | **2.07** × 10⁻¹⁰ |
| FBS | 28 | 21.4 (6/28) | 78.6 (22/28) | 75.0 | 2.42 × 10⁻¹⁰ |

### Example 6: Culturing of cells of various kinds of fish using fish serum

In the present Example, an attempt was made to culture various fish cells in the presence of serum of various kinds of fish.

### Method

Using a cell culture medium (Dulbecco's modified Eagle medium) to which FBS had been added at 10%, cells derived from various kinds of fish were cultured in a closed environment at 25°C (see Table 5-1). The culture supernatant of the cells during culturing was removed, the cells were then washed once with 5 mL of phosphate buffer saline (hereinafter PBS), and 0.334 mL of a trypsin/EDTA solution was added to suspend the cells. To the cell suspension, 5 mL of the cell culture medium was added, and the cells were sufficiently suspended, followed by centrifugation. After the centrifugation, the supernatant was removed. In a 25 cm² flask containing 5 mL of a cell culture medium to which serum derived from any of various kinds of fish shown in Table 5-2 (migratory fish (rainbow trout, eel), freshwater fish (red spotted masu trout, Amur catfish), marine fish (red stingray, spotless smooth-hound, chicken grunt, red seabream, yellowtail, greater amberjack, pacific bluefin tuna)) and treated for inactivation at 45°C for 20 minutes had been added at 0.1 to 1.0%, the cells were seeded at 0.43 × 10⁶ cells/flask. The cells were cultured in a closed environment at 22°C or 25°C, and the cells were confirmed to fix and proliferate. GF cells were also tested with yellowtail serum. SSN-1, GGR-1 and RSB-2 cells were cultured with greater amberjack serum. CHSE-214 cells were cultured with spotless smooth-hound serum, red spotted masu trout serum and rainbow trout serum.

**[Table 5-1]**

| Table 5-1: Type of cultured fish cells, growing environment of fish, and culturing conditions for cells | | | |
|---|---|---|---|
| Name of cell | Origin | Water regime | Culturing temperature |
| CHSE-214 | Chinook salmon fetus | Freshwater/seawater | 22°C |
| RTG-2 | Rainbow trout genital gland | Freshwater/seawater | |
| BF-2 | Bluegill tail | Freshwater | 25°C |
| FHM | Fathead minnow muscle | Freshwater | |
| SSN-1 | Striped snakehead larva | Freshwater | |
| RSB-2 | Red seabream brain | Seawater | |
| GGR-1 | Kuetama brain | Seawater | |
| GF | Chicken grunt fin | Seawater | |

**[Table 5-2]**

| Table 5-2: Origin of serum used | | | |
|---|---|---|---|
| Origin fish | Water regime | Maturity | Farmed/Wild |
| Rainbow trout | Freshwater/seawater | Adult fish | Farmed |
| Eel | Freshwater/seawater | Adult fish | Farmed |
| Red spotted masu trout | Freshwater | Adult fish | Farmed |
| Amur catfish | Freshwater | Juvenile fish | Farmed |
| Red stingray | Seawater | Adult fish | Wild |
| Spotless smooth-hound | Seawater | Adult fish | Wild |
| Chicken grunt | Seawater | Adult fish | Farmed |
| Red seabream | Seawater | Juvenile fish | Farmed |
| Yellowtail | Seawater | Adult fish | Farmed |
| Greater amberjack | Seawater | Juvenile fish | Farmed |
| Pacific bluefin tuna | Seawater | Adult fish | Farmed |

### Results

As in Table 5-3, the fish-derived cells tested were confirmed to be capable of being cultured with all of the fish serum tested. It was confirmed that the cells were capable of being fixed on the culture surface and cultured regardless of whether the living environment relevant to the fish-derived cells and the fish serum was freshwater, seawater or both and whether farmed fish or wild fish. The cells were confirmed to proliferate well even when individuals from which the serum used was derived were mature.

**[Table 5-3]**

| Table 5-3: Culturing results | | | | | | |
|---|---|---|---|---|---|---|
| Name of cell | Fish serum | | | | | |
| | yellowtail | Greater amberjack | Red seabream | Eel | Red stingray | Spotless smooth-hound |
| GF | ++ | ++ | ++ | ++ | ++ | ++ |
| FHM | ND | ND | + | + | + | ++ |
| BF-2 | ND | + | + | ++ | ++ | ++ |
| RTG-2 | ND | + | ++ | + | + | ++ |

| Name of cell | Fish serum | | | | | |
|---|---|---|---|---|---|---|
| | Amur catfish | Chicken grunt | Red spotted masu trout | Rainbow trout | Pacific bluefin tuna | |
| GF | ++ | ++ | ++ | ++ | ++ | |
| FHM | ++ | ++ | ++ | ++ | ++ | |
| BF-2 | + | ++ | ++ | ++ | ++ | |
| RTG-2 | ++ | + | ++ | ++ | ++ | |

| | | | | | | |
|---|---|---|---|---|---|---|
| ++: Good fixation and good proliferation were recognized. +: Fixation and proliferation were recognized. ND: Not done. *: For SSN-1, GGR-1 and RSB-2, the ability to proliferate was confirmed only with greater amberjack serum, good proliferation was recognized (+ and ++). The ability of CHSE-214 to proliferate was ++ with spotless smooth-hound serum, and + with red spotted masu trout and rainbow trout. | | | | | | |

### Example 7: Test of safety and confirmation of effectiveness of red seabream iridovirus oral inactivated vaccine for yellowtail

In the present Example, an oral inactivated vaccine was prepared from a virus proliferated using fish cells, and the effectiveness and the safety of the prepared vaccine for yellowtail were confirmed.

### Method

For confirming that an inactivated iridovaccine (prototype vaccine) was safe for animal subjects, and effective as vaccine against challenge with a red seabream iridovirus, the prototype vaccine was orally administered to juvenile yellowtail to confirm the safety. In addition, an artificial infection test was conducted by injecting a red seabream iridovirus solution to yellowtail inoculated with the prototype vaccine in advance, and the immune effect against the red seabream virus was confirmed.

### Method for preparing prototype vaccine

Formalin was added at a final concentration of 0.2 vol% to a culture solution of red seabream iridovirus-infected GF cells cultured in a DMEM medium using 10% FBS, followed by inactivation at 4°C for 14 days. Thereafter, the inactivated red seabream iridovirus culture containing cells and the culture solution was collected. The inactivated red seabream iridovirus culture was precipitated by centrifugation, and the supernatant was removed to concentrate the culture solution to 1.6 × 10¹¹ TCID₅₀/mL. The thus-obtained concentrate (corresponding to an about 160-fold inactivated virus concentrate) was used as a prototype red seabream iridovirus vaccine.

### Immunization method

The prototype red seabream iridovirus vaccine cultured using 10% FBS was mixed with feed and guar gum as in Table 6-1, and fed for 14 days to the yellowtail farmed in a 100 L tank at 22 fish per tank. As a control group (non-vaccinated group), a food comprising 100% of feed was used instead of the composition of Table 6-1. The prototype vaccine was administered at a dose of 2.2 × 10¹⁰ TCID₅₀/day per fish (that is, about 0.14 mL/day). The control group was fed with only feed. The fish was farmed at a water temperature of 27°C, whether or not there was an abnormal swimming pattern in the farming period was confirmed, and the number of dead fish was counted.

### Challenge method

After completion of the immunization, 0.1 mL of challenging red seabream iridovirus solution BI6 strain was intraperitoneally injected at 4.8 × 10¹ TCID₅₀/fish. After the challenge, the fish was farmed at a water temperature of 27°C for 15 days, and the viability and the mortality in each test group were calculated. For the viability, a significant difference was calculated by the Fisher's exact test method between the test groups and the control group (p < 0.05).

### Results

After the vaccine administration (during immunity period), death did not occur during the immunity period and there was no abnormal finding in clinical observation in any of the test groups (Table 6-2). From this, it was confirmed that oral administration of the prototype virus vaccine to fish was safe for the fish.

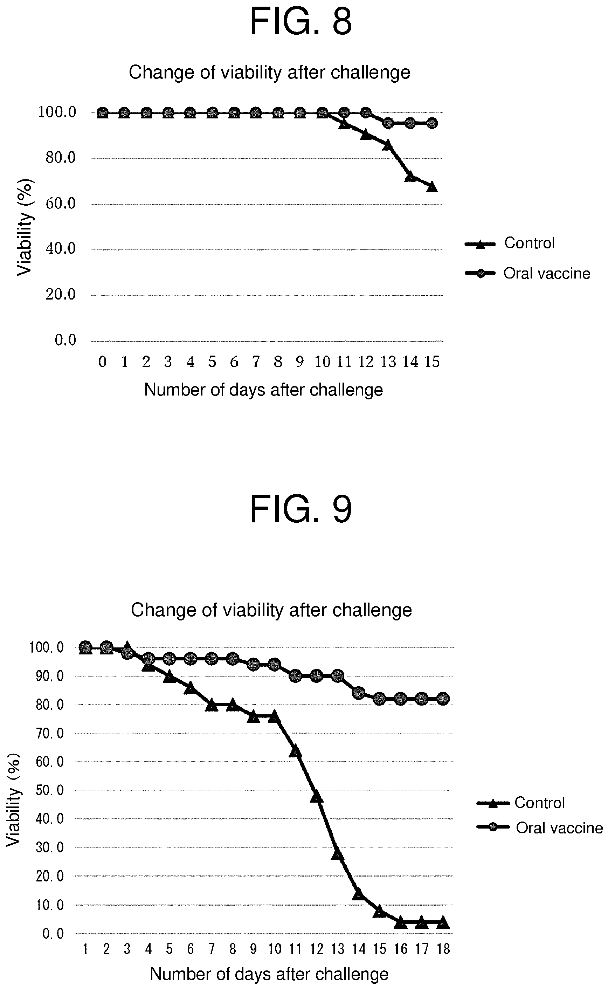

Next, the effect of the vaccination against the challenge was examined. The number of fish died after the challenge is shown in Table 6-3, and a change of the viability is shown in Figure 8. In the control group, death occurred on and after the 11th day of challenge, with the mortality being 31.8% and the viability being 68.2% at the end. On the other hand, the group given oral vaccine had a mortality of 4.5%, a viability of 95.5%, and a vaccine effectiveness percentage of 85.7%. The viability in the group given oral vaccine was confirmed to be significantly higher than the viability in the control group (p < 0.05), and the vaccine was confirmed to be effective. In the present Example, the cells were cultured in the presence of FBS, but adult fish serum may be used instead of FBS.

**[Table 6-1]**

| Table 6-1: Feed containing prototype vaccine | |
|---|---|
| | Proportion (%) |
| Prototype vaccine | 28.6 |
| Feed | 67.4 |
| Guar gum | 4.0 |

**[Table 6-2]**

| Table 6-2: Number of fish died after vaccination | | | | |
|---|---|---|---|---|
| Test group | Number of fish tested (number of fish) | Mortality (%) (dead/tested) | Viability (%) (alive/tested) | Clinical finding |
| Control group | 22 | 0.0 (0/22) | 100.0 (22/22) | No abnormal finding ^{*1} |
| Group given oral vaccine | 22 | 0.0 (0/22) | 100.0 (22/22) | No abnormal finding ^{*1} |

| | | | | |
|---|---|---|---|---|
| *1 No abnormal finding : Abnormality was not observed in the body color, the swimming pattern of fish schools and the feeding behavior in clinical observation during the period of administration. | | | | |

**[Table 6-3]**

| Table 6-3: Number of fish died after challenge | | | | | |
|---|---|---|---|---|---|
| Test group | Number of fish tested (number of fish) | Mortality (%) (dead/tested) | Viability (%) (alive/tested) | Effectiveness percentage (%) | Significant difference (p value; %) |
| Control group | 22 | 31.8 (7/22) | 68.2 (15/22) | - | |
| Group given oral vaccine | 22 | 4.5 (1/22) | 95.5 (21/22) | 85.7 | 4.59 × 10⁻² |

### Example 8: Test of safety and confirmation of effectiveness of red seabream iridovirus oral inactivated vaccine for red seabream

In the present Example, an oral inactivated vaccine was prepared from a virus proliferated using fish cells, and the effectiveness and the safety of the prepared vaccine for red seabream were confirmed.

### Method

For confirming that an inactivated iridovaccine (prototype vaccine) was safe for animal subjects, and effective as vaccine against challenge with a red seabream iridovirus, the prototype vaccine was orally administered to juvenile red seabream to confirm the safety. In addition, an artificial infection test was conducted by immersing red seabream inoculated with the prototype vaccine in advance in seawater to which a red seabream iridovirus solution had been added, and the immune effect against the red seabream virus was confirmed.

### Method for preparing prototype vaccine

Formalin was added at a final concentration of 0.2 vol% to a culture solution of red seabream iridovirus-infected GF cells cultured in a DMEM medium containing 10% FBS, followed by inactivation at 4°C for 14 days. Thereafter, the inactivated red seabream iridovirus culture containing cells and the culture solution was collected. The inactivated red seabream iridovirus culture solution was centrifuged, and the supernatant was removed to concentrate the culture solution to 1.6 × 10¹¹ TCID₅₀/mL. The thus-obtained concentrate (corresponding to an about 160-fold inactivated virus concentrate) was used as a prototype red seabream iridovirus vaccine.

### Immunization method

The prototype red seabream iridovirus vaccine cultured using 10% FBS was mixed with feed and guar gum as in Table 7-1, and fed for 7 days to the red seabream farmed in a 100 L tank at 50 fish per tank. As a control, a food comprising 100% of feed was used instead of the composition of Table 7-1. The prototype vaccine was administered at a dose of 1.0 × 10¹⁰ TCID₅₀/day per fish. The control group was fed with only feed. The fish was farmed at a water temperature of 27°C, whether or not there was an abnormal swimming pattern in the farming period was confirmed, and the number of dead fish was counted. The fish weight was measured at the start of immunization and at the end of immunization.

### Challenge method

After completion of the immunization, the fish was immersed for 1 hour in a virus solution obtained by diluting challenging red seabream iridovirus solution BI6 strain to 0.38 × 10¹ TCID₅₀/L with seawater. After the challenge, the fish was farmed at a water temperature of 25°C for 18 days, and the viability and the mortality in each test group were calculated. For the viability, a significant difference was calculated by the Fisher's exact test method between the test groups and the control group (p < 0.05).

### Results

After the vaccine administration (during immunity period), all the test groups exhibited a good weight gain (Table 7-2). After the vaccine administration, death did not occur during the immunity period and there was no abnormal finding in clinical observation in any of the test groups (Table 7-2). From this, it was confirmed that preparation of a prototype viral vaccine, followed by oral administration of the vaccine to fish, was safe for the fish.

The number of fish died after the challenge is shown in Table 7-3, and a change of the viability is shown in Figure 9. In the control group, death occurred on and after the fourth day of challenge, with the mortality being 96.0% and the viability being 4.0% at the end. On the other hand, the oral vaccine group had a mortality of 18.0%, a viability of 82.0%, and a vaccine effectiveness percentage of 81.3%. The viability in the oral vaccine group was confirmed to be significantly higher than the viability in the control group (p < 0.05), and the vaccine was confirmed to be effective.

**[Table 7-1]**

| Table 7-1: Feed containing prototype vaccine | |
|---|---|
| | Proportion (%) |
| Prototype vaccine | 32.3 |
| Feed | 66.2 |
| Guar gum | 1.5 |

**[Table 7-2]**

| Table 7-2: Average fish body weight and gain of body weight during period of immunization | | | |
|---|---|---|---|
| Test group | Start of immunization | End of immunization | Gain of body weight (g) |
| | Average fish body weight (g) | Average fish body weight (g) | |
| Control group | 3.9 | 4.3 | 0.4 |
| Group given oral vaccine | 3.8 | 4.4 | 0.6 |

**[Table 7-3]**

| Table 7-3: Number of fish died after vaccination | | | | |
|---|---|---|---|---|
| Test group | Number of fish tested (number of fish) | Mortality (%) (dead/tested) | Viability (%) (alive/tested) | Clinical finding |
| Control group | 50 | 0.0 (0/50) | 100.0 (50/50) | No abnormal finding ^{*1} |
| Group given oral vaccine | 50 | 0.0 (0/50) | 100.0 (50/50) | No abnormal finding ^{*1} |

| | | | | |
|---|---|---|---|---|
| *1 No abnormal finding : Abnormality was not observed in the body color, the swimming pattern of fish schools and the feeding behavior in clinical observation during the period of administration. | | | | |

**[Table 7-4]**

| Table 7-4: Number of fish died after challenge | | | | | |
|---|---|---|---|---|---|
| Test group | Number of fish tested (number of fish) | Mortality (%) (dead/tested) | Viability (%) (alive/tested) | Effectiveness percentage (%) | Significant difference (p value; %) |
| Control | 50 | 96.0 (48/50) | 4.0 (2/50) | - | |
| Oral vaccine | 50 | 18.0 (5/50) | 82.0 (45/50) | 81.3 | 4.62 × 10⁻¹⁵ |

### Example 9: Red seabream iridovirus neutralization test

In the present Example, an oral inactivated vaccine was prepared from a virus proliferated using fish cells, followed by confirmation of whether a neutralizing antibody against the red seabream iridovirus was present in the serum of the red seabream to which the prepared vaccine had been administered.

### Method for preparing prototype vaccine

Formalin was added at a final concentration of 0.2 vol% to a culture solution of red seabream iridovirus-infected GF cells cultured in a DMEM medium containing 10% FBS, followed by inactivation at 4°C for 14 days. Thereafter, the inactivated red seabream iridovirus culture containing cells and the culture solution was collected. The inactivated red seabream iridovirus culture solution was centrifuged, and the supernatant was removed to concentrate the culture solution to 1.0 × 10⁹ to 1.6 × 10¹¹ TCID₅₀/mL. The concentrate (corresponding to an about 160-fold inactivated virus concentrate) was used as a prototype red seabream iridovirus vaccine.

### Immunization method and serum collection

The prototype red seabream iridovirus vaccine was administered to the red seabream farmed in a 100 L tank at 3 fish per tank. The prototype vaccine was orally administered at a dose of 4.0 × 10¹⁰ TCID₅₀/day per fish for 3 days in the test class 1, and at a dose of 1.0 × 10¹⁰ TCID₅₀/day per fish for 3 days in the test class 2. In the test class 3, the prototype vaccine was intraperitoneally administered by injection once at 5.0 × 10⁸ TCID₅₀ per fish. In the control class, the vaccine was not administered. The fish was farmed at a water temperature of 25°C for 7 days after the administration. After completion of the immunization, the blood was collected from the red seabream in each test class, the collected blood was centrifuged, and the serum was then collected.

### Neutralization test method

The red seabream iridovirus solution was diluted with a cell culture medium (Dulbecco's modified Eagle medium) to prepare a 2.5 × 10³ TCID₅₀/mL virus solution. The red seabream serum in each test class was incubated at 45°C for 20 minutes to perform inactivation, and then diluted with the cell culture medium to prepare 4-fold diluted serum. Equal amounts of the virus solution and each diluted serum were mixed, and the mixture was incubated at 25°C for 90 minutes, and then incubated at 4°C overnight to obtain a neutralization sample. In a 96-well plate, GF cells were seeded at 7.0 × 10⁵ cells/well, and cultured for 1 day. Each neutralization sample was inoculated in 6 to 9 wells, followed by infection treatment for 24 hours. Thereafter, the cell culture medium was replaced with a new medium for cell culturing (Dulbecco's modified Eagle medium), and the cells were cultured for 6 days. After the culturing, whether there was a cytopathic effect (CPE) or not was confirmed, and the ratio of positive CPE was calculated.

### Results

The ratio of positive CPE in the control class was 83.3%, whereas the ratio of positive CPE in the test classes 1, 2 and 3 was 0% (Table 8). From this, it was confirmed that injection or oral administration of the prototype vaccine to the red seabream produced a neutralizing antibody against the red seabream iridovirus.

**[Table 8]**

| Table 8: Ratio of positive CPE | | | | | |
|---|---|---|---|---|---|
| Test class | Administration method | Dosage (TCID₅₀/day) | Number of dosing days | Number of positive CPE /well | Ratio of positive CPE (%) |
| Test class 1 | Oral | 4.0 × 10¹⁰ | 3 | 0/9 | 0.0 |
| Test class 2 | Oral | 1.0 × 10¹⁰ | 3 | 0/9 | 0.0 |
| Test class 3 | Injection | 5.0 × 10⁸ | 1 | 0/6 | 0.0 |
| Control class | - | - | - | 5/6 | 83.3 |

**[Table 9]**

| Table 9: Summary of test results | | | | | | |
|---|---|---|---|---|---|---|
| | Example 7 Yellowtail | Example 8 Red seabream | Example 9 Neutralized red seabream | | | |
| Immunization method | Oral (feeding) | Oral (feeding) | Test class 1 (oral) | Test class 2 (oral) | Test class 3 (injection) | Control |
| Virus culture solution titer (TCID50/mL) | 1.0E+09 | 1.0E+09 | 1.0E+09 | 1.0E+09 | 1.0E+09 | - |
| Virus concentration ratio (times) | 160 | 160 | 160 | 40 | 1 | - |
| Prototype vaccine concentration (TCID50/mL) | 1.6E+11 | 1.6E+11 | 1.6E+11 | 4.0E+10 | 1.0E+09 | - |
| Dosage per fish (mL/day) | 0.14 | 0.06 | 0.25 | 0.25 | 0.50 | - |
| Dosage per fish (TCI\D50/day) | 2.2E+10 | 1.0E+10 | 4.0E+10 | 1.0E+10 | 5.0E+08 | - |
| Number of dosing days | 14 | 7 | 3 | 3 | 1 | - |
| Number of immunization days (including dosing days) | 14 | 7 | 10 | 10 | 8 | - |
| Challenge method | Injection | Immersion | - | - | - | - |
| Challenge virus titer (TCID50/ml) | 1.6E+05 | 1.3E+04 | - | - | - | - |
| Challenge dose (TCID50/fish) *(TCID50/L) | 4.8E+01 | 3.8E+00 | - | - | - | - |
| Neutralization test (Number of CPE on the sixth day of virus infection/number of wells) | - | - | 0/9 | 0/9 | 0/6 | 5/6 |
| Neutralization test positive ratio | - | - | 0 | 0 | 0 | 83.3 |

Table 9 shows the summary of the results of Examples 7 to 9. Examples 7 and 8 demonstrate that the vaccine for oral administration of the present disclosure was effective, and Example 9 demonstrates that the vaccine for oral administration of the present disclosure induced a neutralizing antibody against the virus in fish individuals.

## Claims

1. An oral inactivated vaccine for fish against a virus infectious disease, comprising an inactivated virus.

2. The oral inactivated vaccine for fish according to claim 1, wherein the virus is an iridovirus.

3. The oral inactivated vaccine for fish according to claim 1 or 2, wherein a concentration of the inactivated virus is 1.0 × 10¹⁰ TCID₅₀/mL to 1.0 × 10¹² TCID₅₀/mL.

4. The oral inactivated vaccine for fish according to any one of claims 1 to 3, wherein an amount of the inactivated virus administered to fish individuals is 0.01 mL/day/fish to 1 mL/day/fish.

5. A method for treating fish, comprising
orally administering to the fish an oral inactivated vaccine for fish against a virus infectious disease, which contains an inactivated virus, which imparts or induces immunity against the virus to the fish.

6. The method according to claim 4, wherein the virus is an iridovirus.

7. The method according to claim 4 or 5, wherein the oral inactivated vaccine for fish is contained in feed.

8. A feed for fish, comprising the oral inactivated vaccine for fish according to any one of claims 1 to **4.**

9. A composition for use in culturing of fish cells, comprising serum obtained from adult fish.

10. A cell culture medium for use in culturing of fish cells, comprising serum obtained from adult fish.

11. A method for culturing fish cells, comprising culturing fish cells in a cell culture medium containing serum obtained from adult fish.

12. The method according to claim 11, wherein a concentration of the serum in the cell culture medium is 0.2% to 2%.

13. A method for culturing a pathogen that infects fish, the method comprising:
providing fish cells infected with the pathogen; and
culturing the fish cells in a cell culture medium containing serum obtained from adult fish to proliferate the pathogen.

14. The method according to claim 13, wherein a concentration of the serum in the cell culture medium is 0.2% to 2%.

15. A method for preparing, from a pathogen that induces an infectious disease in fish, a vaccine against the infectious disease, the method comprising:
providing fish cells infected with a pathogen that induces an infectious disease; and
culturing the fish cells in a cell culture medium containing serum obtained from adult fish to proliferate the pathogen.

16. The method according to claim 15, wherein a concentration of the serum in the cell culture medium is 0.2% to 2%
